# EUROPÄISCHE PATENTSCHRIFT

(11) **EP 0 776 374 B1**
(45) Veröffentlichungstag und Bekanntmachung des Hinweises auf die Patenterteilung: **16.12.2009**
(21) Anmeldenummer: 96920690.3
(22) Anmeldetag: 19.06.1996
(51) Int. Cl.: C12Q 1/00, C12M 1/36

(54) **VERFAHREN UND VORRICHTUNG ZUR BESTIMMUNG DER AKTIVITÄT VON ENZYMEN IN FLÜSSIGKEITEN, BEZIEHUNGSWEISE DER KONZENTRATION UND/ODER AKTIVITÄT VON INHIBITOREN IN FLÜSSIGKEITEN**
PROCESS AND DEVICE FOR DETERMINING THE ACTIVITY OF ENZYMES IN LIQUIDS, OR THE CONCENTRATION AND/OR ACTIVITY OF INHIBITORS IN LIQUIDS
PROCEDE ET DISPOSITIF DE DETERMINATION DE L'ACTIVITE D'ENZYMES DANS DES LIQUIDES OU DE LA CONCENTRATION ET/OU ACTIVITE D'INHIBITEURS DANS DES LIQUIDES

(30) Priorität: 20.06.1995 DE 19522255; 29.07.1995 DE 19527880; 03.05.1996 DE 19617731
(43) Veröffentlichungstag der Anmeldung: 04.06.1997
(73) Patentinhaber: Papst Licensing GmbH & Co. KG, 78112 St Georgen (DE)
(72) Erfinder: Schumacher, Johannes, 69181 Leimen (DE); Werle, Bernd, 96214 eppelheim (DE)
(74) Vertreter: Weber, Roland
(86) Internationale Anmeldenummer: PCT/DE1996/001087
(87) Internationale Veröffentlichungsnummer: WO 1997/000969

(56) Entgegenhaltungen:
- EP-A- 0 019 638
- EP-A- 0 329 190
- EP-A- 0 335 354
- GB-A- 2 213 262
- US-A- 3 983 001
- Koohmaraie; Kretchar (1990) J. Anim. Sci. 68, 2362-2370.
- Anastasi et al: (1983) Biochem. J. 211, 129-138.
- Afting; Becker: (1981) Biochem. J. 197, 519-522
- Schroeder et al: (1977) J. Chromatography 134, 83-90
- de Jong; Kwakman: (1989) J. Chromatography 492, 319-343
- Brinkman et al: (1989) J. Chromatography 492, 251-298

## Beschreibung

Die Erfindung betrifft ein Verfahren und eine Vorrichtung zur Bestimmung der Aktivität von Enzymen in Flüssigkeiten.

Die Bestimmung von Enzymaktivitäten in Pflaczenextrakten, Bakteriensuspensionen, Homogenanten und Körperflüssigkeiten, wie Serum, Plasma, Harn, bronchoalveolärer Lavageflüssigkeit, Punktat, Liquer, Zellinien oder auch homogenisietem Gewebe, hat für die Diagnostik und zur Verlaufs- bzw. Therapiekontrolle ganz wesentliche Bedeutung erlangt.

Beispielsweise in Serum ist die Unterscheidung zwischen Enzymproteinen und den übrigen Serumproteinen auf chemischem Wege äußerst problematisch. Dabei ist zu berücksichtigen, daß die Konzentrationen der einzelnen Enzyme in Körperflüssigkeiten außerordentlich gering sind. So enthält das Serum eines gesunden Menschen 0,1 µg Glutamat-Oxal-Azetat-Transaminase im Milliliter. Zun vergleich sei erwähnt, daß die Gesamteiweißkonzentration 60 bis 80 mg/ml Serum beträgt, d.h. die Konzentrationen verhalten sich etwa wie 1:700.000. Da die Bestimmung der Enzymkonzentrationen auf chemischem Wege problematisch ist, berechnet man ihre Aktivität aus der Geschwindigkeit, mit der die Umsetzung eines geeigneten Substrats erfolgt.

Aus der klinischen Chemie ist das sogenannte ELISA-Verfahren bekannt, bei dem auf immunologischem Wege die Konzentrationen eines Enzyms und des entsprechenden Enzym-Inhibitor-Komplexes in einer Gewebeprobe bzw. in einer Meßprobe erfaßt werden. Eine Messung der Enzymaktivität ist nach diesem Verfahren nicht möglich, da hier lediglich Konzentrationen gemessen werden, wobei nicht berücksichtigt wird, daß ein Enzym sowohl in aktiver als auch in inhibierter Form vorliegen kann.

Aus dem Stand der Technik sind Verfahren bekannt, bei denen einer Meßprobe zunächst die mit einem bestimmten Enzym korrespondierenden Enzym-Inhibitoren entzogen werden und anschließend die Aktivität dieses Enzyms bestimmt wird. Die bekannten Verfahren sind sehr aufwendig, was einerseits auf die verwendeten Meßproben in Form von Gewebeproben zurückzuführen ist und andererseits auf die Vorgehensweise, wie die Enzym-Inhibitoren der Meßprobe entzogen werden. Dazu wird die Meßprobe mit einer Substanz versetzt, die die Enzym-Inhibitoren an sich bindet. Um die Enzym-Inhibitoren möglichst vollständig zu binden, muß die Substanz über eine bestimmte Dauer auf die Meßprobe einwirken, wobei eine möglichst homogene Mischung zwischen Meßprobe und Substanz aufrechterhalten werden muß. Anschließen müssen die so behandelte Meßprobe und die Substanz in einem Trennverfahren separiert werden.

Koohmaraie und Kretschar (1990), J. Anim. Sci. 68, 2362-2370, beschreiben und vergleichen vier Methoden zur Bestimmung der Aktivitäten der lysosomalen Proteasen Cathepsin B und Cathepsin L. In einer der beschriebenen Methoden wird aus einem Muskelhomogenisat ein Extrakt hergestellt und im Batchverfahren mit an Sepharose gekoppeltem Papain für zwei Stunden unter ständigem Mischen inkubiert. Dabei werden die an die Cathepsine gebundenen Inhibitoren in dem Extrakt an das an die Sepharose gekoppelte Papain gebunden. Der so behandelte Extrakt wird dann von dem Sepharosematerial abgetrennt und die Enzymaktivität darin bestimmt. Ein Nachteil dieses Verfahrens besteht darin, dass es eine lange Inkubationszeit und eine Homogenisierung des Materials durch ständiges Mischen erfordert.

Die EP-A-0 329 190 beschreibt ein Verfahren zur Messung einer Enzymaktivität innerhalb einer gepackten Säule, wobei das Säulenmaterial ein fest daran gebundenes Substrat aufweist, das von dem Enzym erkannt wird. Wird die enzymhaltige Lösung auf die Säule aufgebracht, dann wird das an die Säule gebundene Substrat von dem Enzym gespalten und die Spaltprodukte des Substrates im Eluat gemessen. Der Substratsäule kann eine weitere Säule vorgeschaltet sein, welche die Probe vor dem Eintritt in die Substratsäule fraktioniert. Eine Auftrennung eines Enzym-Enzyminhibitor-Komplexes ist nicht vorgesehen.

Die EP-A-0 335 354 beschreibt ein Verfahren und eine Vorrichtung zur Erhöhung der Empfindlichkeit nicht radioaktiver Immunoassays und Nukleinsäurehybridisierungsassays, wobei ein Enzym als Indikator verwendet wird. Zunächst wird ein Komplex aus einem Markerenzym und einem zu bestimmenden Substrat, wie beispielsweise einer Nukleinsäure, gebildet. Das Enzym kann dabei bereits an ein festes Trägermaterial gebunden vorliegen oder nach der Komplexbildung zusammen mit dem Substrat an das Trägermaterial gebunden werden. Es findet dann eine enzymatische Reaktion an dem Substrat statt, und die eluierten Abbauprodukte des Substrates werden anschließend bestimmt, um einen Rückschluß auf ihre ursprüngliche Konzentration vorzunehmen.

Anastasi et al. (1983), Biochem. J. 211, 129-138, beschreiben ein Reinigungsverfahren für Cystatin aus Einweiß, bei dem Ovomucin durch Präzipitation und Zentrifugation entfernt wird, der Überstand einer Affinitätsreinigung des Cystatins an einer Carboxymethyl-Papain-Sepharose-Säule unterzogen wird und das Eluat anschließend einer weiteren chromatographischen Aufreinigung des Cystatins unterzogen wird. Es handelt sich um ein herkömmliches Reinigungsverfahren, jedoch nicht um die Trennung eines Enzym-Enzyminhibitor-Komplexes zur Bestimmung eines der Komplexbestandteile.

Afting und Becker (1981) Biochem. J. 197, 519-522, beschreibe ein Verfahren zur Reinigung von Cathepsin D mit Hilfe einer mit Pepstatin modifizierten Sepharose-Säule. Es handelt sich eine Affinitätsaufreinigung ohne Trennung eines Enzym-Enzyminhibitor-Komplexes. Es ist beschrieben, dass Cathepsin D, welches in Lösung durch 6M Harnstoff irreversibel inaktiviert wird, seine Aktivität an der Pepstatin-Affinitätssäule beibehält, wenn es mit der gleichen Konzentration an Harnstoff gewaschen wird. Die gleiche Aktivität von Cathepsin D wird auch erzielt, wenn die Pepstatin-Sepharose-Säule zuvor nicht mit 6M Harnstoff gewaschen wird. Daraus ziehen die Autoren den Schluß, dass der Abfall der Ausbeute an Cathepsin D nach der Pepstatin-Säule gegenüber einer Concanavalin-Säule nicht auf eine teilweise Inaktivierung des Enzyms durch den Harnstoff zurückzuführen ist, sondern auf die Abtrennung von "Aktivator-Proteinen" in dieser Verfahrensstufe.

Die US-A-3,983,001 beschreibt wieder eine affinitätschromatographische Reinigung eines biologisch aktiven Stoffes, wobei eine diesen biologisch aktiven Stoff bindende Substanz an einen festen Säulenträger kovalent gebunden ist. Eine der Komponenten eines spezifischen Komplexes, wie beispielsweise aus Enzym und Inhibitor oder Antikörper und Antigen, wird kovalent an den festen Träger gebunden, wodurch es möglich wird, die andere Komponente aus der Lösung zu adsorbieren.

Davon ausgehend liegt der Erfindung nun die Aufgabe zugrunde, ein Verfahren und eine Vorrichtung zur Bestimmung der Aktivität von Enzymen in Flüssigkeiten anzugeben, welche eine weitgehende bis vollständige Automatisierung und also eine Rationalisierung der Meßmethode ermöglichen. Gelöst wird die voranstehende Aufgabe durch die Merkmale der Patentanspruch.

Erfindungsgemäß ist erkannt worden, daß die Enzym-Inhibitoren der Meßprobe auch entzogen werden können, ohne daß eine möglichst homogene Mischung zwischen der entsprechenden Substanz und der Meßprobe hergestellt werden muß und anschließend ein aufwendiger Trennvorgang vorgenommen werden muß. In diesem Zusammenhang ist erkannt worden, daß das Mischen und Trennen auch auf chromatographischem Wege aber praktisch in einem Verfahrensschritt durchgeführt werden können. Besonders vorteilhaft daran ist, daß mit dem erfindungsgemäßen Verfahren nunmehr Enzymaktivitäten von in flüssiger Form vorliegenden Meßproben, d.h. von Körperflüssigkeiten aller Art sowie von homogenisierten Gewebeproben, bestimmt werden können, wobei das Entziehen der Enzym-Inhibitoren auf chromatographischem Wege eine weitgehend automatisierte Verfahrensführung ermöglicht.

Dazu wird die Meßprobe in vorteilhafter Weise durch eine Säule mit einem chromatographischen Trägermatieral geleitet, wobei das Trägermaterial mit einer die Enzym-Inhibitoren bindenden Substanz versetzt ist. Dabei reichern sich die Enzym-Inhibitoren in der Säule an, so daß also gleichzeitig, quasi als Nebenprodukt des erfindungsgemäßen Verfahrens auch eine Isolation der Enzym-Inhibitoren erreicht wird.

Um nun die Meßergebnisse verschiedener Meßvorgänge miteinander vergleichen zu können, müssen definierte Versuchsbedingungen eingehalten werden. Dazu kann die behandelte Meßprobe, d.h. die von Enzym-Inhibitoren bereinigte Meßprobe, mit einem geeigneten Säulenpuffer definiert verdünnt werden. Außerdem kann der Meßprobe ein je nach den Versuchsbedingungen und zu bestimmenden Enzymaktivitäten geeigneter Meßpuffer beigemischt werden. In einer vorteilhaften Variante des erfindungsgemäßen Verfahrens läßt sich das Meßassay, d.h. die zumeist verdünnte Meßprobe in Verbindung mit dem Testsubstrat, das durch Einwirken des zu bestimmenden Enzyms in Spaltprodukte aufgespalten wird, während der Inkubationszeit temperieren.

Grundsätzlich gibt es verschiedene Möglichkeiten, um den Anstieg der Konzentration zumindest eines Spaltprodukts des Substrats pro Zeiteinheit während der Inkubationszeit zu erfassen. Besonders vorteilhaft ist es, den Anstieg der Konzentration fluorometrisch zu bestimmen, da sich auf diese Weise besonders gut der Zeitverlauf des Konzentrationsanstiegs verfolgen läßt.

Die erfindungsgemäße Vorrichtung zur Bestimmung der Aktivität von Enzymen in Flüssigkeiten mit den Merkmalen des Patentanspruchs 1 umfaßt eine Säule mit einem chromatographischen Trägermatieral, wobei das Trägermaterial mit einer Substanz versetzt ist, welche in der Meßprobe vorliegende, mit mindestens einem Enzym korrespondierende Enzym-Inhibitoren bindet. Dem einen Ende der Säule ist eine Zuführeinrichtung für Meßproben zugeordnet. In Flußrichtung der Meßprobe, d.h. der Säule nachgeschaltet ist Zweckmäßig eine Ventil/PumpenAnordnung angeordnet, mit der mindestens ein Versuchsgefäß mit einem Substrat und zumindest einem Teil der Meßprobe befüllbar ist. Schließlich ist noch ein Detektor zum Erfassen des Anstiegs der Konzentration zumindest eines Spaltprodukts des Substrats pro Zeiteinheit vorgesehen.

Erfindungsgemäß ist erkannt worden, daß sich ein verfahren zur Bestimmung der Aktivität von Enzymen bzw.Aktivität und/oder Konzentration von Inhibitoren in Flüssigkeiten, bei dem einer Meßprobe auf chromatographischem Wege die mit einem Enzym korrespondierenden Enzym-Inhibitoren entzogen werden, in eine sequentielle Versuchsanordnung umsetzen läßt. Das heißt, eine Meßprobe durchläuft nacheinander verschiedene Stationen einer Versuchsanordnung, ohne daß die Meßprobe vor Beendigung des Versuchs der Versuchsanordnung entnommen werden muß und speziell behandelt werden muß. Besonders vorteilhaft in diesem Zusammenhang ist, daß die Messungen weitgehend automatisch durchgeführt werden können, indem einzelne Komponenten der Vorrichtung automatisch angesteuert und bedient werden.

In einer vorteilhaften Ausgestaltung der erfindungsgemäßen Vorrichtung ist die Säule mehrfach verwendbar, d.h. es lassen sich mit einer Säule mehrere Meßproben hintereinander behandeln. Dazu herrscht in der Säule ein Überschuß an der Substanz, welche die in den Meßproben vorliegenden Enzym-Inhibitoren bindet. Die Menge bzw. der Überschuß an der Substanz, bestimmen letztlich die Kapazität der Säule.

Von Vorteil ist es außerdem, wenn die Säule austauschbar ist. In diesem Falle kann einerseits eine verbrauchte Säule durch eine neue Säule ersetzt werden. Andererseits kann die erfindungsgemäße Vorrichtung dann auch für unterschiedliche Versuche präpariert werden, d.h. für die Messung von Aktivitäten unterschiedlicher Enzyme. Hierfür müssen den Meßproben jeweils unterschiedliche Enzym-Inhibitoren entzogen werden. Dementsprechend muß die Säule also mit unterschiedlichen Substanzen präpariert sein.

Im Hinblick auf eine Automatisierung des Meßverfahrens ist es vorteilhaft, wenn die Zuführeinrichtung nicht nur Zugriff auf einen Meßprobenspeicher, wie z. B. ein Probenrondell, hat sondern auch auf ein Reservoir eines Säulenpuffers. Der Säulenpuffer dient zum Nachspülen bzw. Durchspülen der Säule nach jeder Meßprobe, um ein Vermischen von unterschiedlichen Meßproben in der Säule und also eine Meßwertverfälschung zu vermeiden. Im Rahmen einer automatischen Verfahrensführung wird die Zuführeinrichtung dann also immer abwechselnd auf den Meßprobenspeicher und das Reservoir des Säulenpuffers zugreifen. Vorteilhaft ist es in diesem Zusammenhang auch, wenn der Säule nachgeschaltet eine Kontrollvorrichtung zum Überprüfen der Reinheit des aus der Säule austretenden Säulenpuffers vorgesehen ist. Eine solche Kontrollvorrichtung könnte beispielsweise photometrisch arbeiten oder auch Mittel zur Messung der Leitfähigkeit der aus der Säule austretenden Flüssigkeit umfassen.

Wie bereits erwähnt dient der Säulenpuffer zum Nachspülen der Säule, nachdem eine Meßprobe die Säule durchlaufen hat. Des weiteren dient der Säulenpuffer aber auch zur Verdünnung der Meßprobe. Zur Auswertung der Meßergebnisse und auch um definierte Versuchsbedingungen zu schaffen, ist in einer vorteilhaften Ausgestaltung der erfindungsgemäßen Vorrichtung der Säule nachgeschaltet eine Meßvorrichtung zum Bestimmen der Verdünnung der Meßprobe mit dem Säulenpuffer angeordnet. Eine derartige Meßvorrichtung könnte beispielsweise Mittel zur Volumenbestimmung von Flüssigkeiten umfassen. Da in der Regel das Volumen der Meßprobe bekannt ist, reicht es, das Volumen des hinzukommenden Säulenpuffers zu bestimmen oder das Gesamtvolumen aus Meßprobe und Säulenpuffer.

Je nach Art der Meßprobe und des verwendeten Säulenpuffers kann es vorteilhaft sein auch eine Mischvorrichtung zum Herstellen einer homogenen Mischung der behandelten Meßprobe mit dem Säulenpuffer vorzusehen.

Oftmals ist es auch erforderlich, der Meßprobe, gegebenenfalls dem Säulenpuffer und dem Substrat in dem Versuchsgefäß über die Ventil/Pumpen-Anordnung zusätzlich einen Meßpuffer beizumischen, um definierte Versuchsbedingungen herzustellen. In diesem Zusammenhang erweisen sich auch Mittel zum Temperieren des versuchsgefäßes als vorteilhaft.

Wie bereits im Zusammenhang mit dem erfinäungsgemäßen Verfahren angedeutet, ist es vorteilhaft, wenn der Detektor zum Erfassen des Anstiegs der Konzentration eines Spaltprodukts des Substrats ein Fluorometer umfaßt.

In der Diagnostik ist es oftmals erforderlich, Vergleichsmessungen zwischen den Enzymaktivitäten von unbehandeltem Serum und behandeltem Serum durchzuführen. Dazu ist es vorteilhaft, wenn zwischen der Zuführeinrichtung und der Säule der erfindungsgemäßen Vorrichtung mindestens ein umstellbares Ventil angeordnet ist, über das die Meßprobe alternativ über die Säule oder direkt, ohne Durchlaufen der Säule in das Versuchsgefäß leitbar ist. Auf diese Weise können sowohl Messungen an Meßproben durchgeführt werden, denen die entsprechenden Enzym-Inhibitoren entzogen worden sind, als auch Messungen an unbehandelten Meßproben.

In einer vorteilhaften Weiterbildung der erfindungsgemäßen Vorrichtung ist zusätzlich mindestens ein Ventil vorgesehen, über das zumindest der Säule und der Ventil/Pumpen-Anordnung ein Spülpuffer zum Reinigen zuführbar ist. In vorteilhafter Weise kann so die gesamte Apparatur gereinigt werden.

Schließlich sei noch darauf hingewiesen, daß die erfindungsgemäße Vorrichtung in Verbindung mit einer Rechnereinheit automatisch betrieben werden kann, indem die Rechnereinheit sowohl das Zuführen der Meßproben und gegebenenfalls des Säulenpuffers steuert, als auch die Bestimmung der Verdünnung der Meßprobe und gegebenenfalls das Mischen und das Befüllen der Versuchsgefäße koordiniert. Außerdem kann die Rechnereinheit auch zum Erfassen und Auswerten des Anstiegs der Konzentration eines Spaltprodukts des Substrats pro Zeiteinheit verwendet werden.

Es gibt nun verschiedene Möglichkeiten, die Lehre der vorliegenden Erfindung in vorteilhafter Weise auszugestalten und weiterzubilden. Dazu ist einerseits auf die Patentansprüche, andererseits auf die nachfolgenden Erläuterung von Ausführungsbeispielen der Erfindung anhand der Abbildungen zu verweisen. In Verbindung mit den Erläuterungen der Ausführungsbeispiele der Erfindung werden auch im allgemeinen bevorzugte Ausgestaltungen und Weiterbildungen der Lehre erläutert.

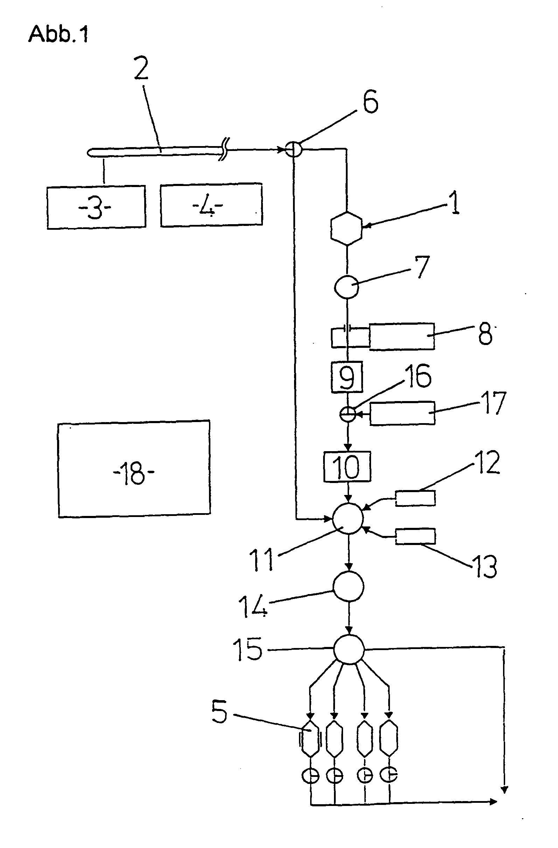

Abb. 1 zeigt in schematischer Darstellung den sequentiellen Aufbau einer erfindungsgemäßen Vorrichtung.

Die in der Abbildung 1 dargestellte Vorrichtung ermöglicht die vollautomatische und serielle Bestimmung der Enzymaktivitäten von verschiedenen in flüssiger Form vorliegenden Meßproben. Dabei kann es sich um Körperflüssigkeiten aller Art oder auch um homogenisiertes Gewebe handeln.

Die Vorrichtung umfaßt erfindungsgemäß zur Behandlung einer Meßprobe eine Säule 1 mit einem chromatographischen Trägermaterial, wobei das Trägermaterial mit einer Substanz versetzt ist, welche in der zu untersuchenden Meßprobe vorliegende, mit mindestens einem Enzym korrespondierende Enzym-Inhibitoren bindet.

Soll beispielsweise die Aktivität des Enzyms Cathepsin H in einer Meßprobe ermittelt werden, so könnte als chromatographisches Trägermaterial der Säule 1 Sepharose-Gel verwendet werden, welches mit der Substanz Papain präpariert ist. Papain bindet die mit Cathepsin H korrespondierenden Enzym-Inhibitoren.

Dem oberen Ende der Säule 1 ist eine Zuführeinrichtung 2 zugeordnet. In dem hier dargestellten Ausführungsbeispiel handelt es sich um einen Entnahmearm 2, der alternativ auf einen Meßprobenspeicher 3 und ein Reservoir 4 für einen Säulenpuffer zugreifen kann.

Der Säule 1 bzw. dem unteren Ende der Säule 1 nachgeschaltet ist eine Ventil/Pumpen-Anordnung, mit der im dargestellten Ausführungsbeispiel mehrere Versuchsgefäße 5 mit einem Substrat und zumindest einem Teil der Meßprobe befüllbar sind.

Zum Erfassen der Aktivität von Cathepsin H in einer Meßprobe kann als Substrat H-Arg-AMC verwendet werden. Durch Einwirken des Enzyms Cathepsin H wird das Substrat H-Arg-AMC in die Spaltprodukte H-Arg und AMC aufgespalten. Da die Enzymaktivität immer proportional zur Konzentration der Spaltprodukte ist, läßt sich mit dem Anstieg der Konzentration zumindest eines Spaltprodukts des Substrats pro Zeiteinheit während einer Inkubationszeit die Enzymaktivität erfassen. Im Falle des Cathepsin H wird der Anstieg der Konzentration von AMC erfaßt. Dazu wird im Rahmen des hier erörterten Ausführungsbeispiels ein Detektor mit einem Fluorometer verwendet, der in der einzigen Figur nicht näher dargestellt ist.

Im folgenden werden nun die einzelnen Komponenten der in der einzigen Figur dargestellten Vorrichtung näher erläutert.

Wie bereits erwähnt sind verschiedene Meßproben in dem Meßprobenspeicher 3, der beispielsweise durch ein Probenrondell gebildet sein kann, angeordnet. Mit Hilfe des Entnahmearms 2 können die Meßproben nun vollautomatisch entnommen werden und über ein Ventil 6 der Säule 1 zugeleitet werden. Das chromatographische Trägermaterial der Säule 1 ist so präpariert, daß in der Säule 1 ein Überschuß an einer Substanz, in der Regel einem oder mehreren Enzymen, herrscht, die die gewünschten Enzym-Inhibitoren der Meßproben an sich binden können. Der Überschuß an dieser Substanz ist in der Regel so groß, daß die Säule 1 mehrfach verwendbar ist, d.h. für den Durchlauf einer größeren Anzahl von Meßproben geeignet ist. Außerdem ist die Säule 1 austauschbar, so daß sie, nach dem die Substanz in der Säule 1 verbraucht ist, durch eine neue Säule ersetzt werden kann. Die Dimensionierung und konstruktive Ausgestaltung der Säule 1 hängt einerseits von der gewünschten Kapazität der Säule 1 ab und andererseits von dem apparativen Umfeld, in dem die Säule 1 angeordnet werden soll. Die Säule 1 kann also beispielsweise mit einem großen Querschnitt und entsprechend kürzer oder auch mit einem kleinen Querschnitt und demtentsprechend länger ausgebildet sein.

Zur Überprüfung der Funktionsfähigkeit der Säule 1 sollten in regelmäßigen Abständen Kontrollmessungen an Meßproben mit bekannter Enzymaktivität durchgeführt werden. Auf diese Weise kann einfach festgestellt werden, ob die Enzym-Inhibitoren bindende Substanz der Säule 1 verbraucht ist.

Um nun zu gewährleisten, daß sich die einzelnen Meßproben nicht miteinander vermischen, kann der Säule 1 alternativ zu einer Meßprobe über den Entnahmearm 2 und das Ventil 6 auch ein Säulenpuffer aus dem Reservoir 4 zugeführt werden. Dies erfolgt immer im Anschluß an das Zuführen einer Meßprobe mit Hilfe einer der Säule 1 nachgeschalteten Pumpe 7.

Dieser Pumpe 7 und also der Säule 1 nachgeschaltet ist eine Kontrollvorrichtung 8 in Form eines Photometers, mit der die Reinheit des aus der Säule 1 bzw. der Pumpe 7 austretenden Säulenpuffers überprüft werden kann. Dadurch wird gewährleistet, daß in der Säule ausschließlich Säulenpuffer vorliegt, bevor der Säule 1 eine weitere Meßprobe zugefüht wird.

Durch das Nachspülen der Säule 1 mit Säulenpuffer wird die Meßprobe, die die Säule 1 bereits durchlaufen hat, verdünnt. Der Verdünnungsgrad wird nun in einer der Säule 1 ebenfalls nachgeschalteten Meßvorrichtung 9 im Rahmen einer Volumenmessung bestimmt. Dabei ist das Volumen der Meßprobe bekannt. Erfaßt wird lediglich das Volumen des zum Spülen verwendeten Säulenpuffers. Aus dem Gesamtvolumen von Meßprobe und Säulenpuffer kann nun der Verdünnungsgrad berechnet werden.

Der Meßvorrichtung 9 nachgeschaltet ist eine Mischvorrichtung 10, mit Hilfe derer die Mischung aus Meßprobe und Säulenpuffer homogenisiert wird.

Die dabei entstehende homogene Mischung wird einem Ventil 11 zugeführt, über das sowohl diese Mischung als auch das Substrat aus einem Substratbehälter 12 und ein Meßpuffer aus einem entsprechenden Meßpufferbehälter 13 den Versuchsgefäßen 5 zugeleitet werden können. Der Meßpuffer 13 dient dabei zur Herstellung definierter Versuchsbedingungen. Alternativ oder zusätzlich können der Mischung auch weitere Lösungen, beispielsweise eine Titerlösung in Form einer speziellen Inhibitorlösung zur Konzentrationsbestimmung, zugesetzt werden. Hierfür muß die Vorrichtung dann zusätzlich entsprechende Behälter umfassen. Dem Ventil 11 sind in dem hier dargestellten Ausführungsbeispiel noch eine Pumpe 14 und ein weiteres Ventil 15 nachgeschaltet, die der Weiterleitung des nunmehr vorliegenden Gemischs aus Meßprobe, Säulenpuffer, Substrat und Meßpuffer dienen.

Die gesamte hier beschriebene Vorrichtung läßt sich temperieren. In der Regel erfolgt die Behandlung der Meßprobe zum Entziehen der Enzym-Inhibitoren bei einer Temperatur von ca. 4 °C. Insbesondere sind Mittel zur Temperierung der Versuchsgefäße 5 vorgesehen, die in der einzigen Figur allerdings nicht dargestellt sind. Die Versuchgefäße sollten zumindest während der Inkubationszeit temperierbar sein. Standardgemäß arbeitet man bei Temperaturen von ca. 37 °C. Im Rahmen spezieller Versuchsbedinungen könnte aber auch eine flexible Temperierung sinnvoll sein. Die Inkubationszeit, d.h. die Einwirkzeit der Meßprobe auf die Substrate, hängt von den unterschiedlichen Enzymen und Substraten ab und liegt in der Regel zwischen 5 und 15 Minuten.

An dieser Stelle sei angemerkt, daß es mit der erfindungsgemäßen Vorrichtung möglich ist, einerseits redundante Messungen durchzuführen, indem in mehreren Versuchsgefäße die Aktivität ein und desselben Enzyms einer Meßprobe bestimmt wird. Andererseits ist es bei entsprechender Präparation der Säule 1 auch möglich, die Aktivitäten unterschiedlicher Enzyme einer Meßprobe zu bestimmen, indem unterschiedliche Substrate verwendet werden, man die behandelte Meßprobe also auf unterschiedliche Substrate einwirken läßt. Denkbar wäre schließlich auch noch die Bestimmung der Aktivitäten eines Enzyms auf unterschiedlichen Substraten.

Mit der in der Abbildung 1 dargestellten Vorrichtung können auch Vergleichsmessungen durchgeführt werden, indem Meßproben durch die Säule 1 in die Versuchsgefäße 5 geleitet werden können oder über das Ventil 6, das Ventil 11, die Pumpe 14 und das Ventil 15 direkt in die Versuchgsgefäße 5 geleitet werden können. Parallel zu den Messungen an Meßproben, die die Säule 1 durchlaufen haben, können also auch Messungen der Enzymaktivität an Meßproben vorgenommen werden, die die Säule 1 nicht durchlaufen haben. Damit sind sogenannte Vorher-/Nachher-Vergleichsmessungen möglich, nämlich Messungen bei Gegenwart von Enzym-Inhibitoren in der Meßproben und Messungen an von Enzym-Inhibitoren, bereinigten Meßproben.

Die in der Abbildung 1 dargestellte Vorrichtung umfaßt schließlich noch ein Ventil 16 über das der gesamten Apparatur ein Spülpuffer 17 zum Reinigen der Apparatur zuführbar ist.

Mit Hilfe einer Rechnereinheit 18 lassen sich die einzelnen Komponenten der erfindungsgemäßen Vorrichtung ansteuern, so z.B. die Zuführeinrichtung, so daß der Säule 1 automatisch Meßproben und Säulenpuffer zugeführt werden können. Auch die Auswertung der Meßergebnisse kann mit Hilfe der Rechnereinheit 18 erfolgen.

Insgesamt läßt sich mit Hilfe des erfindungsgemäßen Verfahrens und der erfindungsgemäßen Vorrichtung eine rationelle, zumindest weitgehend automatische Meßmethode zur Bestimmung der Enzymaktivitäten von in flüssiger Form vorliegenden Meßproben realisieren.

In der klinisch-chemischen und mikrobiologischen Diagnostik sowie in der biochemischen Forschung hat die Bestimmung von Enzymaktivitäten einen wichtigen Stellenwert. Zur Durchführung solcher Bestimmungen ist bereits eine große Zahl von Verfahren bekannt und zur routinemäßigen Anwendung gelangt.

Dennoch war es bisher unmöglich, die Aktivitäten verschiedener Enzyme schnell und kostengünstig zu messen, da viele von ihnen im Serum, in anderen Körperflüssigkeiten oder in ihren Uraprungszellen zum Schutz ihrer Umgebung teilweise oder vollständig inhibiert vorliegen, d.h. sie sind dort, wo sie Schaden anrichten können, zum überwiegenden Teil inaktiv.
Kommt es nun zu einer pathologischen Veränderung der Umgebung oder der Ursprungszellen selbst, kann sich das Verhältnis zugunsten der nicht inhibierten, aktiven Form ändern, wobei besonders im Serum ein dadurch entstehender Enzymüberschuß möglichst umgehend durch Inhibitoren reversibel blockiert wird.
Daher ist es mit bisher zur Anwendung gelangten Methoden nur bei außergewöhnlich hohen Enzymkonzentrationen möglich, beispielsweise im Serum die freie Aktivität der nicht inhibierten Enzyme zu messen.
Zur frühzeitigen Diagnose von Erkrankungen kann es jedoch notwendig sein, einen Anstieg der absoluten Enzymaktivität pro Volumeneinheit oder das Verhältnis der Volumenaktivitäten der inhibierten zur freien Enzymfraktion möglichst schnell und sicher zu messen, da die mit herkömmlichen Methoden meßbaren Spitzenaktivitäten der freien Enzymfraktion oft erst in fortgeschrittenem Krankheitsverlauf auftreten.
Auch in der Forschung kann die Kenntnis des aktiven Anteils eines Enzyms am Gesamtpool von Bedeutung sein.

Mittels immunologischer Methoden (ELISA) kann zwar die Konzentration auch des inhibierte Anteils einer Enzymklasse bestimmt werden, jedoch ist ein bedeutender Nachteil der immunologischen Methoden die Unmöglichkeit der Unterscheidung zwischen freiem, also aktivem, und inhibiertem Enzym, da ein solches Verfahren die Summe beider Gruppen sowie auch teilweise oder vollständig funktionsuntüchtige Enzyme erfaßt. Eine Messung von Aktivitäten ist ausgeschlossen.

Mittels bekannter Assays zur Messung von Enzymaktivitäten können die beiden Zustandsformen der Enzyme ebenfalls nicht differenziert werden, da hierbei lediglich die freie Form gemessen werden kann.

Wie kann nun zusätzlich zur freien Aktivität eines Enzyms, das in inhibiertem und freiem Zustand vorliegen kann, die Gesamtaktivität gemessen werden?
Die Lösung ist einfach und kostengünstig:
Der Probe, beispielsweise humanem Serum, werden auf chromatographischem Wege spezifische Inhibitoren, die das gewünschte Enzym in seiner Aktivität hemmen, entzogen. Anschließend kann eine normale Aktivitätsmessung durchgeführt werden.
Wird zusätzlich die Aktivität des Enzyms in der unbehandelten Probe gemessen, kann der Anteil des freien bzw. inhibierten Enzyms an der Gesamtfraktion dieses Enzyms in der Probe bestimmt werden.

Das folgende Beispiel soll der Veranschaulichung des Verfahrens dienen:
An ein Sepharose-Gel als Trägersubstanz wird die Cystein-Proteinase Papain gekoppelt. Das so präparierte Gel wird in eine Chromatographiesäule gepackt. Einer in dieser Säule inkubierten Probe, z.B. Gewebehomogenat aus einem Lungentumor, werden alle Inhibitoren entzogen, die eine höhere Affinität zu Papain als zu ihren ursprünglichen Enzymen aufweisen. Diese Enzyme können unter anderem die Cathepsine 5, H und L sein, wobei Cathepsin H normalerweise nahezu vollständig inhibiert vorliegt und seine Enzymaktivität erst nach Entzug der Inhibitoren (Stefin A, Kininogen und andere) meßbar wird.
Die Messung der Aktivitäten erfolgt nach den bekannten Assays auf flourometrischem Wege unter Verwendung enzymspezifischer Substrate und Inhibitoren.

Dieses Verfahren, das je nach Verwendungszweck mit den unterschiedlichsten Chromatographiematerialien, Enzymen und Inhibitoren durchführbar ist, kann vollständig automatisiert werden und ist damit für den Routineeinsatz im Labor geeignet (siehe hierzu Abbildung 1).
So kann eine große Anzahl an Proben auf einem Probenrondell oder in einem anderen geeigneten Meßprobenspeicher (3) gelagert werden, wovon jeweils ein definiertes Volumen der zu messenden Probe durch eine Entnahmevorrichtung (2) mittels einer nachgeschalteten Pumpe (7) in eine geeignete Chromatographiesäule (1) geführt wird.
Nach einer vorgeschriebenen Inkubationszeit wird die von den gewünschten Inhibitoren gereinigte Probe durch eine auf die gleiche Weise eingeführte definierte Pufferlösung aus einem Säulenpufferreservoir (4) vollständig aus der Säule gespült und gleichzeitig verdünnt.
Eine anschließende Reinheitsmessung (8), beispielsweise auf photometrischem Wege oder durch Messung der Leitfähigkeit, schließt Probenrückstände in der Säule aus und verhindert gleichzeitig einen unnötig hohen Pufferverbrauch.
Mittels einer weiteren Meßeinrichtung (9) kann das aus der Säule gespülte Gesamtvolumen bestimmt werden.
Um eine homogene Mischung des Probe-Puffergemischs nach dem Ausspülen aus der Säule zu erreichen, ist dieser eine MischVorrichtung (10) nachgeschaltet.
Mittel einer weiteren Pumpe kann über ein Ventil, das wie alle anderen Funktionseinheiten des Geräts zentral von einem Rechner (18) angesteuert wird, in der vorgeschriebenen Reihenfolge Meßpuffer, Substrat, eventuell Inhibitoren und die in einem bekannten Verhältnis durch die anfangs verwendete Pufferlösung verdünnte Probe in eine flourometrische Meßeinheit (5) geleitet werden.
Der Rechner kann nun aus den Meßdaten auf einfach Weise die in der Originalprobe vorliegende Aktivität des gewünschten Enzyms pro Volumeneinheit oder, bei Kenntnis der Proteinkonzentration in diesem Volumen, die Aktivität pro Masseeinheit errechnen.
Zum Vergleich der solchermaßen gemessenen Enzymaktivität mit der Aktivität in der unbehandelten Probe kann diese unter Umgehung der Chromatographiesäule auch direkt zur Messung gebracht werden.
Nach oder bereits während dem Meßvorgang kann mit der automatisch gesteuerten Spülung der Vorrichtung begonnen werden, um Rückstand der Probe vollständig zu entfernen.
Weitere Erläuterungen zu Abbildung 1 finden sich in der Stammanmeldung.

Um spezielle Aufgaben mit der beschriebenen Vorrichtung optimal lösen zu können, kann die Anordnung einiger Einheiten, insbesondere der Ventile, von der in Abbildung 1 gezeigten abweichen.

Vorteilhaft ist es, wenn zur Steigerung der Effizienz der Vorrichtung mehrere Säulen parallel oder in Reihe geschaltet sind. Auch mehrere parallele flourometrische Messungen der Aktivitäten unterschiedlicher Enzyme aus derselben Probe tragen zu einer höheren Leistungsstärke der Vorrichtung bei.

Anstatt flourometrischer Messung der Aktivitäten spezifischer Enzyme kann auch, je nach Bedarf und verwendetem Meß-Assay, ein anderes Meßsystem, z.B. ein photometrisches, verwendet werden.

Als Besonderheit können unterschiedlich präparierte, selbstverständlich mehrfach verwendbare Säulen verwendet werden, so beispielsweise mit unterschiedlichen Enzymen oder großtechnisch hergestellten Enzymfragmenten sowie auch mit Inhibitoren bestückte Chromatographie-Materialien.

Dank dieser zahlreichen Möglichkeiten können neben Enzymen auch, je nach Säule, Inhibitoren gereinigt werden.
Wird eine mit Enzymen präparierte Säule verwendet, können die den Proben entzogenen Inhibitoren nach dem Erreichen der Kapazität der Säule durch ein einfaches chemisches Verfahren von den Enzymen getrennt und der weiteren Verwendung zugeführt werden. Auf diese Weise könnten auch bislang unbekannte Inhibitoren angereichert und klassifiziert werden.

Die Abbildungen erläutern die Weiterbildung.

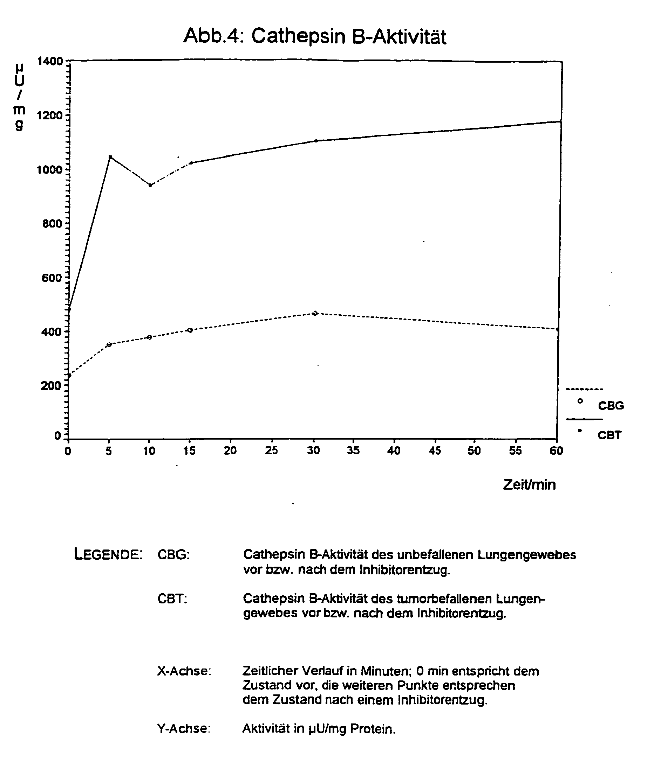

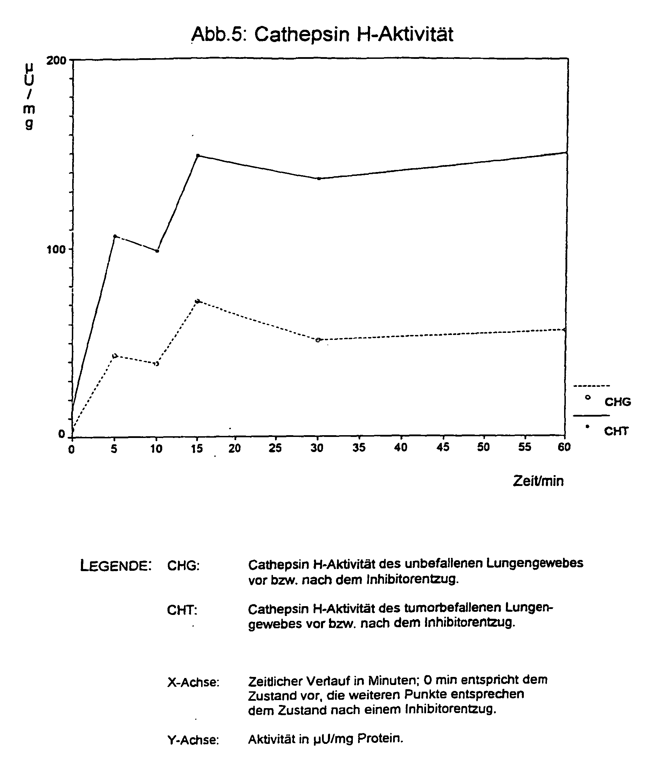

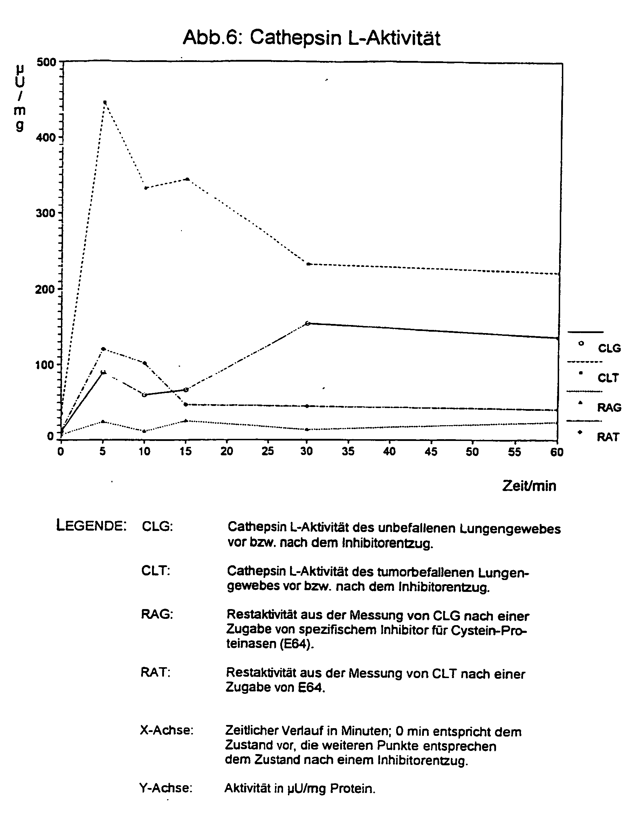

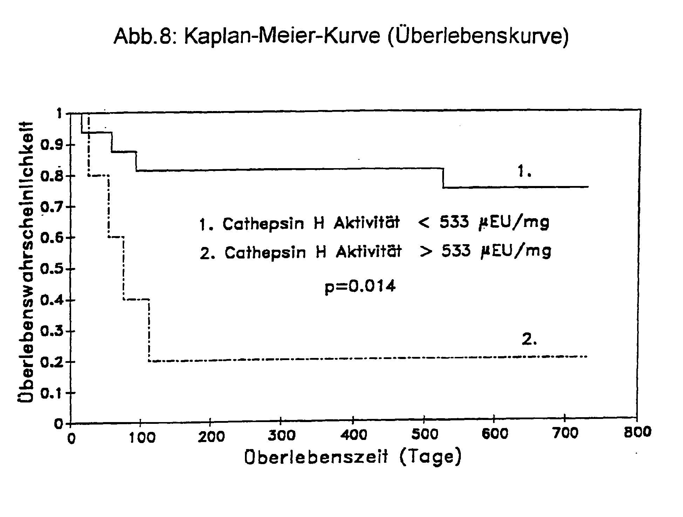

Die Effizienz der oben beschriebenen Papainsäule (Sepharose-Gel als chromatographisches Tragermaterial, Papain als daran gekoppeltes Enzym) wird im Zusammenhang mit der Untersuchung der Proteinasen Cathepsin B, H und L durch die folgenden Messungen beispielhaft belegt:
1. Gemessen wurden die Aktivitäten (µU/mg Protein) der Cathepsine B, H und L in Lungengewebehomogenaten von Tumorpatienten vor und nach einem Entzug der korrespondierenden Inhibitoren durch ein mit Papain präpariertes Sepharose-Gel. Die Inkubationszeit der Proben in der Säule wurden auf 15 Minuten festgelegt.

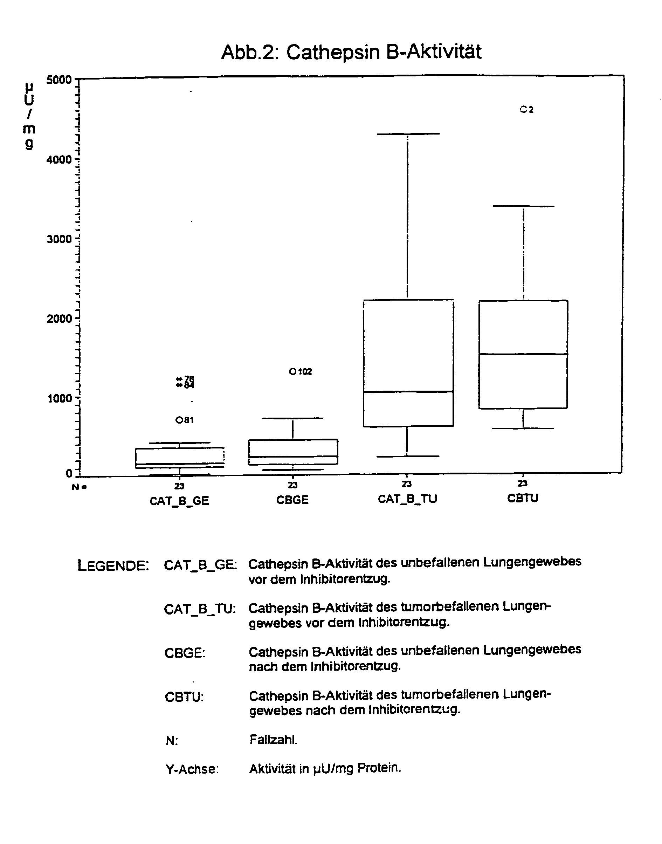

   Abbildung 2 zeigt, daß die Aktivität sowohl im unbefallenen als allen im tumorbefallenen Lungengewebe nach einem Entzug der Inhibitoren auf den Säulen im Median wesentlich höher als in der unbehandelten Probe liegt.
   In dieser Grafik wurde für den Vorher-/Nachher-Vergleich jeweils dasselbe Probenkollektiv verwendet. Der Anstieg der Aktivitäten ist nur durch einen Entzug der Inhibitoren zu erklären und literaturkonform.

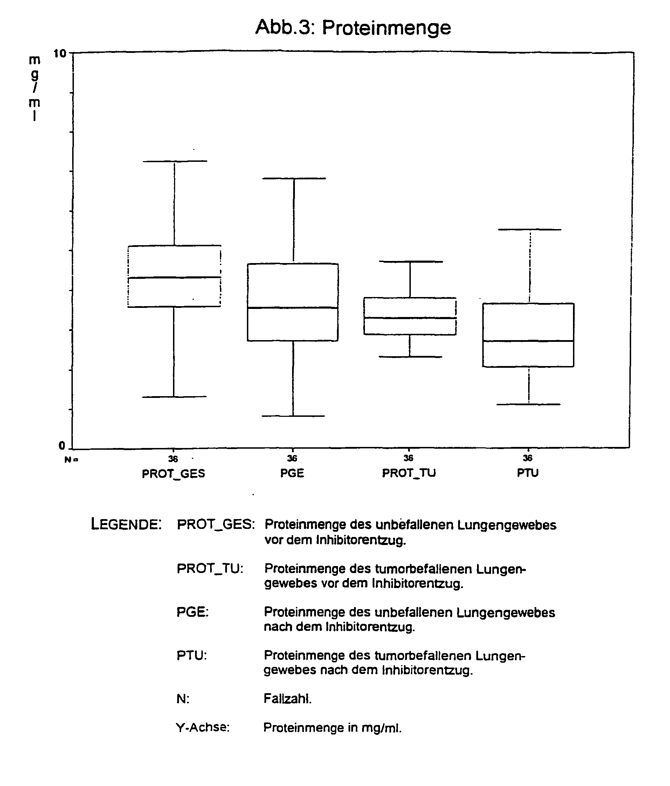

2. Abbildung 3 zeigt, daß vor einem Entzug der Inhibitoren auf der Papainsäule die Proteinmenge (mg/ml) im Median wesentlich höher liegt als nach einem Entzug, was nur durch einen tatsächlich stattfindenden Entzug der Inhibitoren zu erklären ist.
3.Als Beispiel für den zeitlichen Verlauf der Inhibitorabgabe an das auf die Säule gebundene Papain wurde ein Probenpaar zufällig ausgewählt und die Aktivitäten nach jeweils unterschiedlich langen Inkubationszeiten auf der Papainsäule bestimmt.
   Aus Abbildung 4 ist ersichtlich, daß die Aktivität von Cathepsin B vom Zeitpunkt 0 an rapide steigt, um nach etwa 15 Minuten in ein Plateau überzugehen. Der Zeitpunkt 0 ist identisch mit der Messung vor einem Inhibitorentzug.
   Auch Abbildungen 5 und 6 zeigen einen starken Anstieg der Aktivitäten nach kurzer Inkubationszeit. Die in Abbildung 6 genannte Restaktivität aus der Aktivitätsmessung von Cathepsin L ist die Aktivität eines noch nicht näher klassifizierten Enzyms.

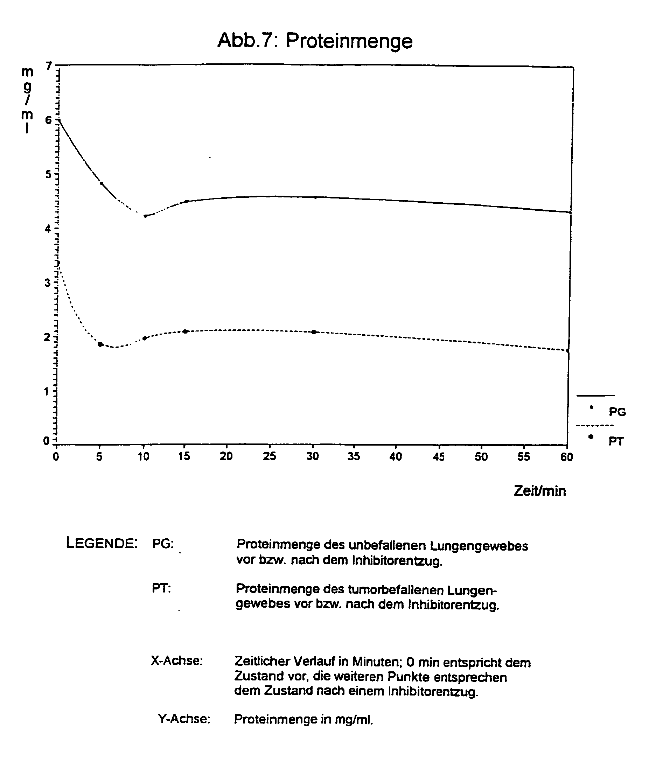

4.Abbildung 7 stellt den Verlauf der Abnahme des Proteingehalts der unter 3. beschriebenen Probe dar. Schon nach kurzer Zeit hat die Proteinmenge ihr Minimum erreicht und geht im zeitliches Verlauf in ein Plateau über; d.h. die Inhibitoren werden dank des Überschusses an Papain in der Säule schnell und sicher gebunden.
5. Aus der in Abbildung 8 gezeigten Überlebenskurve (Kaplan-Meier-Kurve) ist ersichtlich, daß diejenigen Patienten, deren Cathepsin H-Aktivität im Tumorgewebe über einem Schwellenwert von 533µEU/mg liegt, eine wesentlich ungünstigere Prognose bezüglich der Überlebenszeit haben als diejenigen, deren Wert unter diesem Schwellenwert liegt. Die Werte für diese wie auch die oben genannten Abbildungen stammen aus Originalmessungen, die mittels einer Papainsäule durchgeführt wurden.

Aus den oben genannten Ausführungen ist -beispielsweise- zu folgern:
- In vielen Körperflüssigkeiten, z.B. Serum, Urin oder Liquor, wie auch in Gewebehomogenaten, Bakteriensuspensionen und anderen Flüssigkeiten liegen in ihrer Aktivität durch spezifische Inhibitoren blockierte Enzyme vor. Die Aktivität dieser Enzymen konnte bisher nicht oder nur bei einem Vorliegen von Spitzenwerten gemessen werden.
- Immunologische Verfahren wie beispielsweise das relativ teure ELISA-Prinzip können nur Gesamtkonzentrationen, nicht jedoch Aktivitäten von intakten und somit funktionstüchtigen Enzymen bestimmen.
- Durch die Verwendung einer entsprechend präparierten chromatographischen Säule ist es möglich geworden, der Probe spezifische Inhibitoren zu entziehen und somit Gesamt- und Teilaktivitäten eines oder mehrerer Enzyme zu messen.
- Ein Nebeneffekt dieser Methode ist die Reinigung und Anreicherung spezifischer Inhibitoren auf der Säule. Diese können anschließen zu Klassifizierungszwecken von dem chromatographischen Material getrennt werden.
- Dank einer Automatisierung kann die oben beschriebene oder eine den Bedürfnissen entsprechende, modifiziert Vorrichtung im täglichen Routinebetrieb des klinisch-chemischen Labors, in der mikrobiologischen Diagnostik oder auch in der biochemischen Forschung eingesetzt werden.
- Dank der Mehrfachverwendbarkeit der Chromatographiesäulen und ihren niedrigen Herstellungskosten ist es möglich, in kurzer Zeit eine große Anzahl von Proben sehr kostengünstig zu untersuchen.
- Durch die Einführung einer entsprechenden Vorrichtung wird ein bisher mangels geeigneter Technik unbearbeitetes Aufgaben- und Forschungsgebiet erschlossen.
- Dank des beschriebenen Verfahren und der Vorrichtung ist es möglich, eine Prognose über den Krankheitsverlauf und die Überlebenszeit der Patienten zu erstellen und die Patienten somit einer ihren speziellen Bedürfnissen angepaßte Therapie zuzuführen. Es wird Aufgabe der Forschung sein, mittels des Verfahrens und der Vorrichtung neue, aussagefähige Tumormarker und Verlaufsparameter herauszuarbeiten.
- Auch für die Bakteriologie und Mikrobiologie kann das Verfahren und die Vorrichtung von Bedeutung sein, da die biochemische Klassifikation von beispielsweise Bakterien von beträchtlicher Bedeutung für die Diagnostik ist.

Da die oben beschriebene Vorrichtung nur in gewissem Umfang transportabel ist und sich zur Durchführung eines preisgünstigen Schnelltests außerhalb des Labors wenig eignet, ist, aufbauend auf dem oben beschriebenen und in Abbildung 1 gezeigten Verfahren, die nachfolgend beschriebene Vorrichtung zur Durchführung eines Schnelltests von Vorteil.

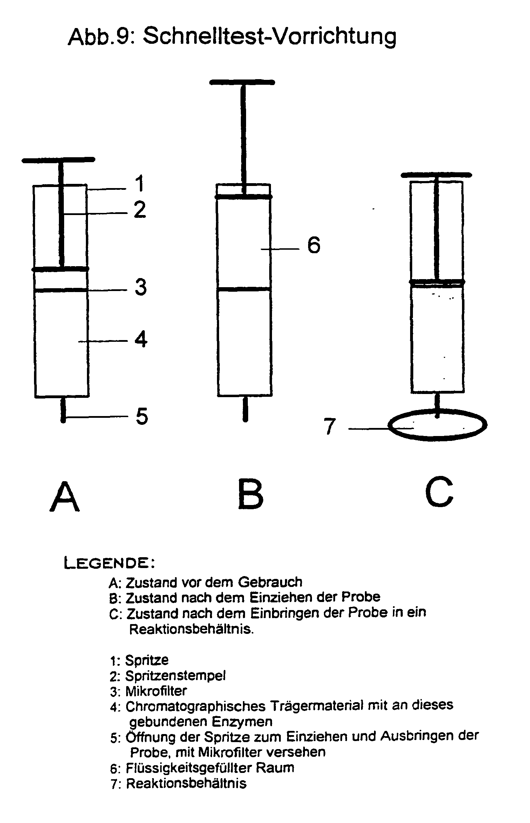

Abbildung 9 erläutert die Weiterbildung.

In einer speziellen Spritze (1), die schematisch in den Zustandsformen A,B und C dargestellt ist, befindet sich im unteren Teil zwischen zwei Mikrofiltern (3 sowie Bestandteil von 5), ein chromatographisches Material (4), an das analog zum oben beschriebenen Verfahren Enzyme, Inhibitoren oder großtechnisch hergestellte Fragmente gekoppelt sind.

Im Zustand vor dem Gebrauch (A) befindet sich im selben Raum wie das chromatographische Material sowie über diesem, jedoch unterhalb des Spritzenstempels (2) im flüssigkeitsgefüllten Raum (6) eine den Erfordernissen entsprechende Flüssigkeit, beispielsweise eine Spülpufferlösung.

Um den Reaktionszustand (B) zu erreichen, wird eine Probe, beispielsweise Blut, Serum, Urin, Liquor oder eine andere Flüssigkeit, durch einen mittels des Spritzenstempels (2) hergestellten Unterdruck durch die Spritzenöffnung (5), die mit einem Mikrofilter versehen ist, in den Raum, in dem sich das chromatographische Material befindet, eingezogen. Daran schließt sich eventuell eine definierte Inkubationszeit an. Im Reaktionszustand werden der Probe je nach chromatographischem Material die Inhibitoren, z.B. Kininogene bei Verwendung eines mit Papain bestückten Sepharose-Gels, oder die Enzyme entzogen.

Danach wird die so behandelte Probe mittels Druck auf den Spritzenstempel mitsamt der sich in Zustand A über dem Filter (3) und gleichzeitig unter dem Spritzenstempel (2) befindenden Flüssigkeit aus der Spritze durch die Spritzenöffnung (5) hindurch in ein Reaktionsbehältnis (7) verbracht (C).

In dem Reaktionsbehältnis kann sich ein spezifisches Substrat befinden, das beispielsweise durch solchermaßen von ihren Inhibitoren befreite Enzyme spezifisch gespalten wird und auf diese Weise, ähnlich einem Indikator, die Farbe verändert.

Nach einer definierten Zeit kann, beispielsweise anhand der Farbveränderung im Reaktionsbehältnis, das Ergebnis des Schnelltests abgelesen werden.
Dank dieses Verfahrens ist es möglich, auch ohne ein aufwendiges Laborgerät, welchem die in Abbildung 1 beschriebene Vorrichtung zugrundeliegt, eine entsprechende Diagnostik und Prognostik, wenn auch nicht mit den Möglichkeiten und der Präzision, die ein solches Laborgerät bietet, durchzuführen. Zur Anwendung gelangen könnte ein solcher Schnelltest beispielsweise in der präklinischen wie auch klinischen Notfalldiagnostik sowie in der allgemeinärztlichen Praxis.

## Patentansprüche

1. Vorrichtung zur Bestimmung der Aktivität von Enzymen in einer flüssigen Meßprobe, die wenigstens ein Enzym und wenigstens einen zu dem Enzym korrespondierenden Enzyminhibitor enthält, wobei die Vorrichtung folgendes umfaßt:
eine Durchlauf-Chromatographiesäule (1), die ein darin gepacktes festes Trägermaterial und eine an das Trägermaterial gebundene Substanz enthält, welche in der Lage ist, den wenigstens einen Enzyminhibitor zu binden,
ein umstellbares Ventil (6), über das die Meßprobe oder Säulenpuffer alternativ über die Durchlauf-Chromatographiesäule (1) oder über eine Bypass-Leitung an der Durchlauf-Chromatographiesäule (1) vorbei geführt werden kann,
einen Meßprobenspeicher (3) und ein Säulenpufferreservoir (4) und einen Detektor zum Erfassen des Anstiegs der Konzentration zumindest eines Spaltprodukts pro Zeiteinheit,
wenigstens ein Versuchsgefäß (5) zur Aufnahme von Meßprobe und ggf. damit gemischtem Puffer und/oder Substrat, wobei das Versuchsgefäß temperierbar ausgestaltet ist,
und wenigstens eines der folgenden der Durchlauf-Chromatographiesäule nachgeschalteten Elemente:
eine Kontrollvorrichtung (8) zur Überprüfung der Reinheit der aus der Durchlauf-Chromatographiesäule austretenden Flüssigkeit,
eine Meßvorrichtung (9) zum Bestimmen der Verdünnung der Meßprobe mit Säulenpuffer,
eine Mischvorrichtung (10) zum Herstellen einer gleichmäßigen Mischung der Meßprobe mit zudosiertem Säulenpuffer,
einen Substratbehälter (12) und/oder einen Meßpufferbehälter (13) in Verbindung mit einem Ventil (11) für das Zudosieren von Substrat und/oder Meßpuffer zu eluierter flüssiger Meßprobe aus der Durchlauf-Chromatographiesäule (1).

2. Vorrichtung nach Anspruch 1, wobei die Vorrichtung weiterhin eine der Durchlauf-Chromatographiesäule (1) nachgeschaltete Ventil/Pumpen-Anordnung (7, 11, 14, 15) für die Beförderung der Meßprobe zu wenigstens einem Versuchsgefäß (5) und für die Zudosierung von Substrat, Säulenpuffer und/oder Meßpuffer zu der Meßprobe umfaßt.

3. Vorrichtung nach einem der Ansprüche 1 oder 2, wobei die Meßvorrichtung (9) Mittel zur Volumenbestimmung von Flüssigkeiten umfaßt.

4. Vorrichtung nach einem der Ansprüche 1 bis 3, wobei die Kontrollvorrichtung (8) ein Photometer oder eine Einrichtung zur Messung der Leitfähigkeit in einer Flüssigkeit umfaßt.

5. Vorrichtung nach einem der Ansprüche 1 bis 4, wobei weiterhin eine Rechnereinheit (18) vorgesehen ist zum automatischen Betreiben und Steuern zumindest der Zuführung der Meßproben und ggf. des Säulenpuffers, ggf. der Bestimmung der Verdünnung der Meßprobe und ggf. des Mischens, des Befüllens des Versuchsgefäßes (5) oder der Versuchsgefäße (5) und des Erfassens und Auswertens des Anstiegs der Konzentration zumindest eines Spaltprodukts des Substrats pro Zeiteinheit.

6. Verfahren zur Bestimmung der Aktivität von Enzymen in einer flüssigen Meßprobe, die wenigstens ein Enzym und wenigstens einen zu dem Enzym korrespondierenden Enzyminhibitor enthält, unter Benutzung der Vorrichtung gemäß den Ansprüchen 1 bis 5.

7. Verfahren gemäß Anspruch 6, wobei man in dem Verfahren
a) eine Durchlauf-Chromatographiesäule bereitstellt, die ein darin gepacktes festes Trägermaterial und eine an das Trägermaterial gebundene Substanz enthält, wobei die Substanz in der Lage ist, den wenigstens einen Enzyminhibitor zu binden,
b) die flüssige Meßprobe in die Chromatographiesäule einbringt und mit der an das Trägermaterial gebundenen Substanz in der Chromatographiesäule in Kontakt bringt, um der Meßprobe den wenigstens einen Enzyminhibitor zu entziehen,
c) die gemäß Stufe b) behandelte, aus der Chromatographiesäule eluierende flüssige Meßprobe sammelt,
d) wenigstens einen Teil der gesammelten Meßprobe aus Stufe c) mit einem Substrat für das Enzym, dessen Aktivität zu bestimmen ist, umsetzt
e) die Konzentration an Umsetzungs- oder Abbauprodukt des durch die Wirkung des Enzyms pro Zeiteinheit umgesetzten Substrates bestimmt.

## Claims

1. A device for measuring the activity of enzymes in a liquid sample that includes at least one enzyme and at least one enzyme inhibitor corresponding to this enzyme, the device comprising:
a flow through column (1) filled with a solid chromatographic carrier and a substance bound to this chromatographic carrier which is capable of binding the at least one enzyme inhibitor,
a switchable valve (6) by means of which the sample or the column buffer can be routed alternatively through the flow through column (1) or through a bypass past the flow through column (1),
a sample reservoir (3) and a column buffer reservoir (4) and a detector for recording the increase of the concentration per time unit of at least one cleavage product,
at least one reaction vessel (5) for receiving the sample and if necessary buffer mixed therewith and/or substrate, whereby the reaction vessel is temperature-controlled,
and at least one of the following elements downstream of the flow through column:
a control device (8) for checking the clarity of the liquid discharged from the flow through column,
a measuring device (9) for determining the degree of dilution of the sample due to added column buffer,
a mixing device (10) for creating a constant mixture of the sample with the added column buffer,
a substrate reservoir (12) and/or a measuring buffer reservoir (13) combined with a valve (11) for adding substrate and/or measuring buffer to the liquid sample eluted from the flow through column (1).

2. A device according to claim (1) whereby in addition this device includes a valve/pump arrangement (7, 11, 14, 15) downstream of the flow through column (1) so as to discharge the sample to at least one reaction vessel (5) and for adding substrate, column buffer and/or measuring buffer to the sample.

3. A device according to one of the claims 1 or 2, whereby the measuring device (9) includes means for determination of the volume of liquids.

4. A device according to one of the claims 1 to 3, whereby the control device (8) includes a photometer or a device for measuring the conductance of a liquid.

5. A device according to one of the claims 1 to 4, whereby in addition a computer unit (18) is provided for automation and control of at least the feeding of the samples and if necessary the addition of the column buffer and if necessary of the determination of the degree of dilution of the sample and if necessary of the mixing, of the feeding of the reaction vessel (5) or the reaction vessels (5) und for recording and analysing the increase of the concentration per time unit of at least one cleavage product of the substrate.

6. A process for measuring the activity of enzymes in a liquid sample that includes at least one enzyme and at least one enzyme inhibitor corresponding to this enzyme by means of using the device according to claims 1 to 5.

7. A process according to claim 6, whereby in this process
a) a flow through column is provided filled with a solid chromatographic carrier and a substance bound to this chromatographic carrier which is capable to bind the at least one enzyme inhibitor,
b) the liquid sample is fed into the chromatographic column so that it gets in contact with the substance bound to the chromatographic carrier of the chromatographic column in order to withdraw the at least one enzyme inhibitor from the sample,
c) the liquid sample which is treated according to stage b) and eluted from the chromatographic column is collected,
d) at least one part of the sample collected according to stage c) reacts with a substrate of the enzyme the activity of which has to be measured,
e) the concentration per time unit is determined of the reaction or degradation product of the substrate resulting from the enzyme catalysed reaction.

## Revendications

1. Dispositif destiné à déterminer l'activité d'enzymes dans un échantillon liquide pour essai comportant au moins un enzyme et au moins un inhibiteur d'enzyme correspondant à l'enzyme, le dispositif comportant :
une colonne de chromatographie en continu (1) contenant un matériau de support solide remplissant ladite colonne et une substance liée au matériau de support, laquelle est en mesure de lier au moins un inhibiteur d'enzyme,
une soupape (6) réversible par l'intermédiaire de laquelle l'échantillon pour essai ou un tampon de colonne peut être guidé par la colonne de chromatographie en continu (1) ou par une conduite de dérivation en passant devant la colonne de chromatographie en continu (1),
un réservoir d'échantillon pour essai (3) et un réservoir de tampon de colonne (4) et un détecteur destiné à détecter l'augmentation de la concentration d'au moins un produit de décomposition par unité de temps,
au moins un récipient d'essai (5) destiné à recevoir l'échantillon pour essai et éventuellement le tampon et/ou le substrat mélangé à celui-ci, le récipient pour essai étant conçu de manière à pouvoir être tempéré,
et au moins un des éléments placés à la suite de la colonne de chromatographie en continu :
un dispositif de contrôle (8) destiné à procéder à la vérification de la pureté du liquide sortant de la colonne de chromatographie en continu,
un dispositif de mesure (9) destiné à déterminer la dilution de l'échantillon pour essai avec le tampon de colonne,
un dispositif de mélange (10) destiné à fabriquer un mélange homogène de l'échantillon pour essai avec le tampon de colonne ajouté,
un récipient de substrat (12) et/ou un récipient de tampon de mesure (13) en liaison avec une soupape (11) pour l'ajout d'un substrat et/ou d'un tampon d'essai à ajouter à l'échantillon pour essai liquide élué de la colonne de chromatographie en continu (1).

2. Dispositif selon la revendication 1, le dispositif comportant en outre un système soupape/pompe (7, 11, 14, 15) monté en aval de la colonne de chromatographie en continu (1) pour acheminer l'échantillon pour essai vers au moins un récipient d'essai (5) et pour ajouter le substrat, le tampon de colonne et/ou le tampon de mesure à l'échantillon pour essai.

3. Dispositif selon l'une quelconque des revendications 1 ou 2, le dispositif de mesure (9) comportant des moyens destinés à déterminer le volume des liquides.

4. Dispositif selon l'une quelconque des revendications 1 à 3, le dispositif de contrôle (8) comportant un photomètre ou un système de mesure de la conductivité dans un liquide.

5. Dispositif selon l'une quelconque des revendications 1 à 4, une unité de calcul (18), destinée au fonctionnement automatique et à la commande d'au moins l'amenée des échantillons pour essai et éventuellement du tampon de colonne, éventuellement de la détermination de la dilution de l'échantillon pour essai et éventuellement du mélange, du remplissage du récipient d'essai (5) ou des récipients d'essai (5) et de la détection et de l'analyse de l'augmentation de la concentration d'au moins un produit de décomposition du substrat par unité de temps, étant prévue.

6. Procédé de détermination de l'activité d'enzymes dans un échantillon liquide pour essai contenant au moins un enzyme et au moins un inhibiteur d'enzyme correspondant à l'enzyme, au moyen du dispositif selon l'une quelconque des revendications 1 à 5.

7. Procédé selon la revendication 6, comprenant les étapes consistant à
a) mettre à disposition une colonne de chromatographie en continu contenant un matériau de support solide remplissant ladite colonne et une substance liée au matériau de support, la substance étant en mesure de lier au moins un inhibiteur d'enzyme,
b) introduire l'échantillon liquide pour essai dans la colonne de chromatographie et le mettre en contact avec la substance liée au matériau de support dans la colonne de chromatographie, afin de prélever le ou les inhibiteurs d'enzyme de l'échantillon pour essai,
c) collecter l'échantillon liquide pour essai traité selon l'étape b) et élué de la colonne de chromatographie,
d) décomposer au moins une partie de l'échantillon pour essai collecté lors de l'étape c) avec un substrat pour l'enzyme dont l'activité est à déterminer,
e) déterminer la concentration de produit de décomposition du substrat décomposé par unité de temps en raison de l'action de l'enzyme.
